# FASCICULE DE BREVET EUROPEEN

(11) **EP 3 539 037 B1**
(45) Date de publication et mention de la délivrance du brevet: **14.06.2023**
(21) Numéro de dépôt: 17804932.6
(22) Date de dépôt: 13.11.2017
(51) Int. Cl.: G16B 15/30, G16B 30/10

(54) **METHODE DE PREDICTION DE LA RECONNAISSANCE CROISEE DE CIBLES PAR DES ANTICORPS DIFFERENTS**
VERFAHREN ZUR VORHERSAGE DER KREUZERKENNUNG VON ZIELEN DURCH VERSCHIEDENE ANTIKÖRPER
METHOD FOR PREDICTING THE CROSS-RECOGNITION OF TARGETS BY DIFFERENT ANTIBODIES

(30) Priorité: 14.11.2016 FR 1660940
(43) Date de publication de la demande: 18.09.2019
(73) Titulaire: Institut national de recherche pour l'agriculture, l'alimentation et l'environnement, 75007 Paris (FR); Centre National de la Recherche Scientifique, 75016 Paris (FR)
(72) Inventeur: BOURQUARD, Thomas, Houston 77006 (US); MUSNIER, Astrid, 37000 Tours (FR); POUPON, Anne, 41100 Mazange (FR); CREPIEUX, Pascale, 37540 Saint Cyr Sur Loire (FR); REITER, Eric, 37380 Nouzilly (FR); BRUNEAU, Gilles, 37380 Reugny (FR)
(74) Mandataire: Cabinet Becker et Associés
(86) Numéro de dépôt international: PCT/FR2017/053083
(87) Numéro de publication internationale: WO 2018/087494

(56) Documents cités:
- US-A1- 2011 224 913
- SEFID FATEME ET AL: "Homology modeling of a Camelid antibody fragment against a conserved region ofAcinetobacter baumanniibiofilm associated protein (Bap)", JOURNAL OF THEORETICAL BIOLOGY, ACADEMIC PRESS, LONDON, GB, vol. 397, 18 février 2016 (2016-02-18), pages 43-51, XP029496858, ISSN: 0022-5193, DOI: 10.1016/J.JTBI.2016.02.015
- Elias Egho ET AL: "On measuring similarity for sequences of itemsets", Data Mining and Knowledge Discovery, 1 mai 2015 (2015-05-01), pages 732-764, XP055393711, Boston DOI: 10.1007/s10618-014-0362-1 Extrait de l'Internet: URL:https://hal.archives-ouvertes.fr/file/ index/docid/740231/filename/RR-8086.pdf [extrait le 1977-02-27]

## Description

### Domaine de l'invention

La présente invention est relative au domaine de la biologie, en particulier des anticorps.

### Arrière-Plan de l'invention

Les méthodes classiques de développement d'un anticorps consistent à immuniser un animal, puis à récupérer son sérum pour obtenir des anticorps polyclonaux ou, pour obtenir des anticorps monoclonaux, à fabriquer des hybridomes à partir de ses plasmocytes, ou bien encore à cribler une banque de phage préparée à partir des ARNm des plasmocytes de l'animal immunisé (phage display).

Les anticorps ainsi obtenus sont ensuite testés *in vitro,* puis chez l'animal, puis enfin chez l'homme. Le développement d'un tel anticorps thérapeutique coûte plusieurs millions d'euros, et prend plusieurs années.

Ainsi, il serait extrêmement utile de pouvoir identifier les potentielles réactions croisées des anticorps afin d'anticiper leurs possibles effets secondaires et toxicité.

*A contrario,* lorsque le développement d'un anticorps thérapeutique aboutit à une impasse due à des propriétés physico-chimiques de l'anticorps défavorables, par exemple faible solubilité, mauvaise stabilité, toxicité, ou difficulté de production, il serait souhaitable de disposer d'une alternative plus rapide que de reprendre le développement de l'anticorps depuis le début.

Il existe des méthodes de modélisation de la structure 3D d'anticorps comme décrit par Sefid Fateme et al. (2016), J. Theoret. Biol., vol. 397, p. 43-51. Cependant, les méthodes décrites sont généralement lourdes à mettre en oeuvre et aucune ne permet de prédire une reconnaissance croisée de cibles entre plusieurs anticorps.

Ainsi, émerge le besoin d'une méthode permettant d'identifier parmi des anticorps connus ceux qui sont capables de reconnaître les mêmes cibles que des anticorps de référence, cette méthode devant être suffisamment facile à mettre en oeuvre pour permettre le criblage d'une large banque d'anticorps de séquence protéique connue.

### Résumé de l'invention

Les inventeurs ont mis au point une méthode permettant d'identifier efficacement, parmi une banque de données d'anticorps de séquence protéique connue, un ou des anticorps qui présentent la même spécificité antigénique qu'un anticorps de référence.

La présente invention concerne une méthode permettant d'identifier des anticorps capables de se lier à la même cible, la méthode comprenant :
- la fourniture d'une base de données comprenant la caractérisation d'au moins 100 anticorps, chaque anticorps étant caractérisé par une séquence simplifiée et une structure secondaire des CDRs (Région Déterminante Complémentaire) CDR1, CDR2 et CDR3 pour chaque domaine variable d'un anticorps de la base de données et ;
   - la séquence simplifiée étant une séquence traduite où chaque acide aminé de la séquence initiale des CDRs est remplacé par un code représentant une catégorie d'acides aminés, les catégories étant basées sur les caractéristiques physico-chimiques des acides aminés et le nombre de catégories étant compris entre 4 et 10 ; et
   - la structure secondaire étant une séquence traduite où chaque acide aminé de la séquence initiale des CDRs est remplacé par un code représentant un type de structure secondaire, le nombre de types de structure secondaire étant compris entre 3 et 8 ;
- la fourniture d'une séquence simplifiée et une structure secondaire des CDRs CDR1, CDR2 et CDR3 pour un domaine variable d'un anticorps de référence ;
- le calcul d'un score de similitude entre le domaine variable de l'anticorps de référence et un domaine variable d'un anticorps test de la base de données sur la base de la séquence simplifiée de leurs CDRs et de la structure secondaire de leurs CDRs par identification de sous-séquences communes aux CDRs de l'anticorps de référence et de l'anticorps test; et
- la sélection de l'anticorps test si le score de similitude est tel qu'il prédit que l'anticorps test et l'anticorps de référence sont capables de se lier à la même cible, l'anticorps test sélectionné étant appelé anticorps similaire.

Dans un mode de réalisation préféré, les catégories basées sur les caractéristiques physico-chimiques des acides aminés sont choisies parmi la taille des acides aminés, leur hydrophobicité, leur polarité, leur charge, leur caractère aromatique et des combinaisons de ces caractéristiques. Par exemple, les catégories choisies sont :
- La petite taille pour les acides aminés A, G, S, T, C et P ;
- Le caractère Aromatique pour les acides aminés Y, F et W ;
- L'hydrophobicité pour les acides aminés I, L, F, M et V ;
- La Polarité pour les acides aminés N et Q;
- La charge positive pour les acides aminés H, K et R ; et
- La charge négative pour les acides aminés D et E.

Dans un mode de réalisation préféré, les types de structure secondaire sont choisis parmi les suivants ou sont les suivants :
- hélice 3₁₀ ;
- hélice α ;
- hélice π;
- coude fermé ;
- brin β;
- résidu dans un pont β;
- coude; et
- une absence de structure secondaire.

Dans un mode de réalisation préféré, la méthode comprend en outre une étape de validation *in vitro* de la capacité de liaison de l'anticorps similaire à la cible de l'anticorps de référence ou de l'anticorps de référence à la cible de l'anticorps similaire.

Dans un mode de réalisation préféré, la méthode comprend une étape de validation *in vitro* de la capacité de liaison de l'anticorps similaire à la cible de l'anticorps de référence. Cette étape de validation peut comprendre la fourniture ou la production de l'anticorps similaire, sa mise en contact avec la cible de l'anticorps de référence, et la mesure de la liaison entre l'anticorps similaire et la cible de l'anticorps de référence. De préférence, les CDR1, CDR2 et CDR3 sont greffés dans un squelette de nano-anticorps. La méthode peut comprendre une étape de sélection de l'anticorps similaire si la capacité de liaison de l'anticorps similaire à la cible de l'anticorps de référence est validée *in vitro.*

Dans un mode de réalisation préféré, la méthode comprend une étape de validation *in vitro* de la capacité de liaison de l'anticorps de référence à la cible de l'anticorps similaire. Cette étape de validation peut comprendre la production ou la fourniture de l'anticorps de référence, sa mise en contact avec la cible de l'anticorps similaire, et la mesure de la liaison entre l'anticorps de référence et la cible de l'anticorps similaire. Cette étape de validation peut comprendre une étape de sélection de l'anticorps de référence si la capacité de liaison de l'anticorps de référence à la cible de l'anticorps similaire est validée *in vitro.*

Dans un mode de réalisation particulier, les anticorps sont des nano-anticorps.

### Description des Figures

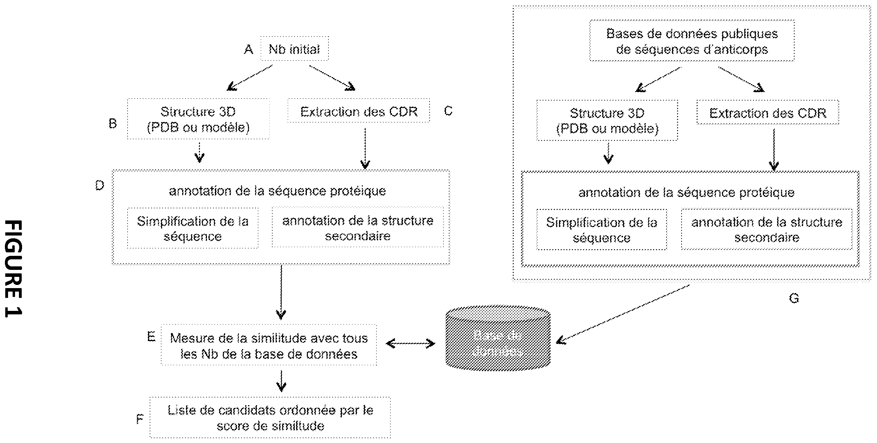

**Figure 1****. Schéma général de la méthode.** A : En entrée, on dispose d'un anticorps initial ou anticorps de référence (Nb initial). Si c'est un anticorps divalent, les domaines variables de la chaîne lourde (VH) et de la chaîne légère (VL) sont considérés comme des nano-anticorps (Nb) et traités séparément. B : Soit la structure 3D est disponible dans la Protein Data Bank (PDB), soit on réalise un modèle par homologie. C : extraction des CDRs. D : recodage par simplification de la séquence protéique et annotation des structures secondaires. E : calcul de la similitude du Nb initial avec tous les Nb de la base de données. F : liste de candidats ordonnée par le score de similitude. G : la base de données utilisée est constituée de séquences de Nb, correspondant à des anticorps monovalents ou à des VH ou VL d'anticorps divalents, dont la structure 3D est soit connue, soit modélisée par homologie, et qui sont recodée de la même manière que le Nb initial.

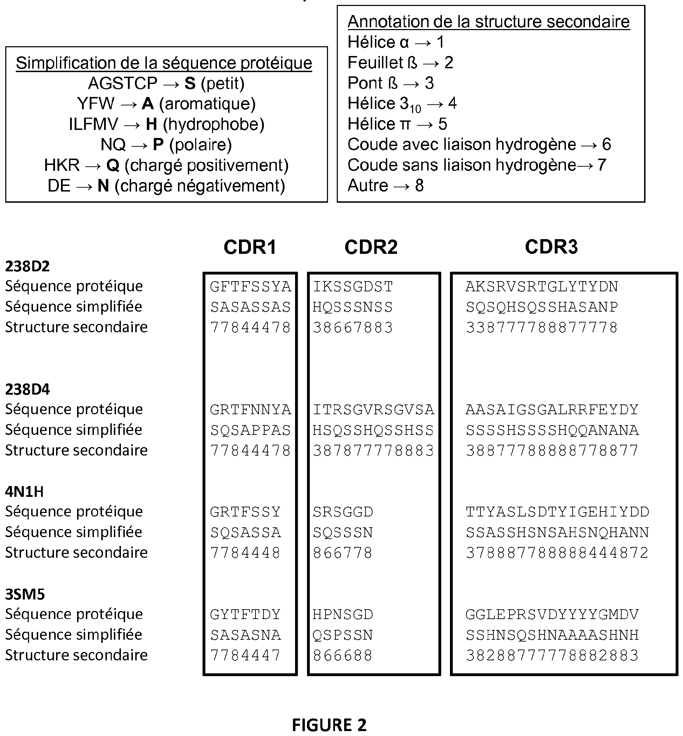

**Figure 2****. Principe de recodage des séquences de CDR.** Simplification et annotation des séquences de Nb. Séquences simplifiées et annotées des CDRs des Nb considérés dans les exemples.

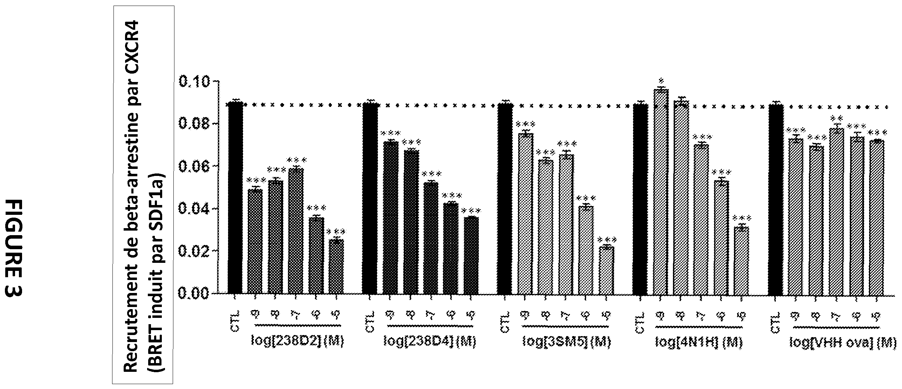

**Figure 3****. Effets des anticorps sur le recrutement des β-arrestines.** Des cellules HEK293 ont été transitoirement transfectées avec le CXCR4 fusionné à la RLuc8 et la β-arrestine 2 fusionnée à la YFP. Quarante-huit heures après la transfection, les cellules sont exposées aux différents VHH aux concentrations indiquées pendant 2 heures à 37°C en PBS-Hepes 10 mM. Elles ont ensuite été stimulées avec le SDF1 à 50 nM. Le signal BRET est lu 15 minutes après la stimulation et après ajout de la Coelenterazine H à 5 µM. Les résultats sont exprimés en moyennes ± sem de n>3 expériences indépendantes réalisées en triplicats.

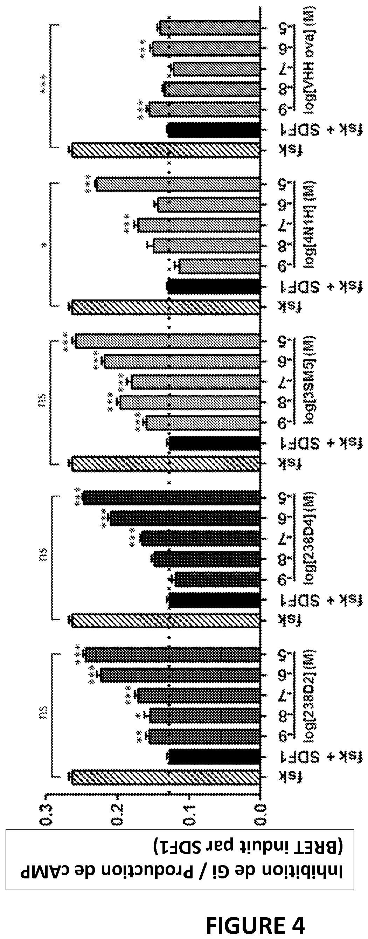

**Figure 4****. Inhibition de la voie Gi par les anticorps.** Des cellules HEK293 ont été transitoirement transfectées avec le CXCR4 et le senseur AMPc CAMYEL. Quarante-huit heures après la transfection, les cellules sont exposées aux différents VHH aux concentrations indiquées pendant 2 heures à 37°C en PBS-Hepes 10 mM. Elles ont ensuite été stimulées avec le SDF1 à 50 nM. Le signal BRET est lu 15 minutes après la stimulation et après ajout concomitant de la Coelenterazine H à 5 µM et de la forskoline à 100 µM. Les résultats sont exprimés en moyennes ± sem de n>3 expériences indépendantes réalisées en triplicats.

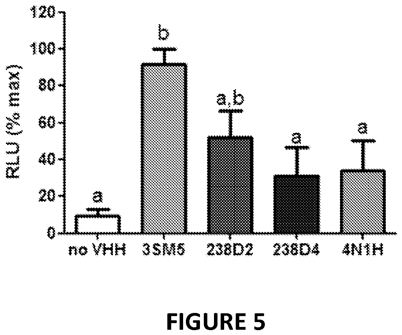

**Figure 5****. Liaison de 3SM5, 238D2, 238D4 et 4N1H à l'hémagglutine (H1N1), cible de 3SMS.** Les surfaces des plaques ELISA sont chargées avec l'hémaglutinine H1N1 à 2 µg/m! et surchargées en PBS-lait 1%. Les VHH ont été incubés 2 heures à température ambiante à 1 µM en PBS et l'anticorps secondaire anti-c-myc-HRP a été incubé au 1:1000 1h à température ambiante. La révélation est faite en ECL. Les résultats sont exprimés en moyennes ± sem du pourcentage de la réponse maximale obtenue dans chacune des 5 expériences réalisées en triplicat.

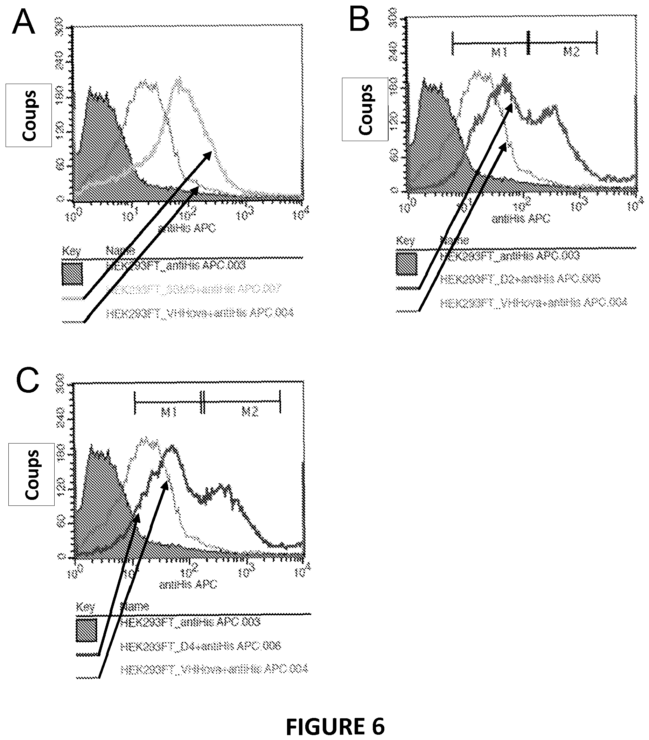

**Figure 6**. 3SM5 lie le CXCR4, comme le 238D2 et le 238D4. Des cellules HEK293 ont été transitoirement transfectées avec le CXCR4. Quarante-huit heures après la transfection, les cellules sont exposées aux VHH 3SM5 (A), 238D2 (B), 238D4 (C), et ovalbumin (A, B, C) à 1 µM pendant 1 heure à température ambiante. La liaison des VHH aux cellules est révélée à l'aide d'un anticorps anti-His couplé à l'allophycocyanin (APC). La fluorescence est mesurée au FACSCalibur.

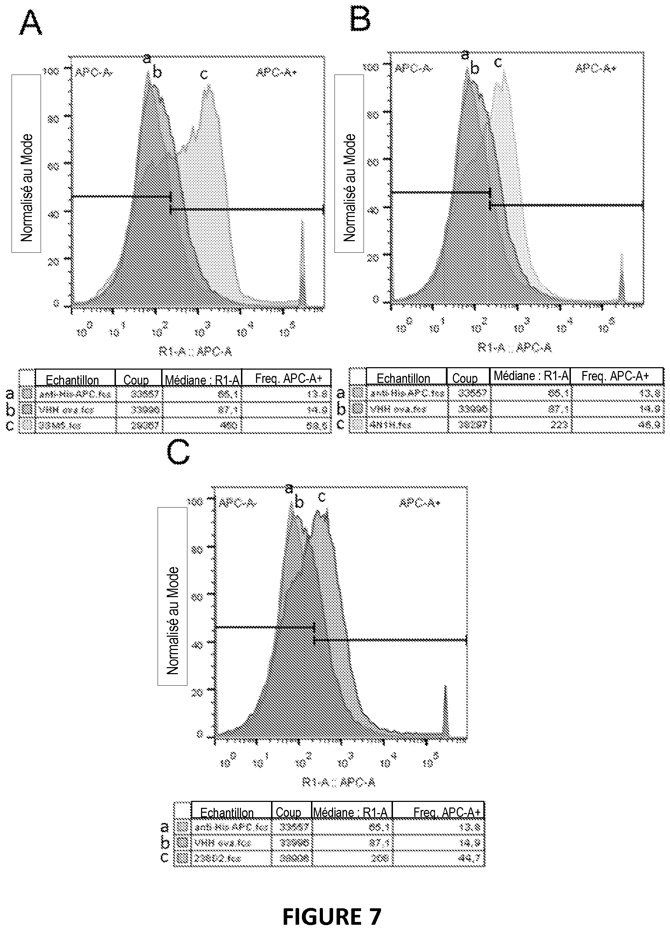

**Figure 7**. 3SM5 et 4N1H lient le CXCR4 comme le 238D2. Des cellules HEK293 ont été transitoirement transfectées avec le CXCR4. Quarante-huit heures après la transfection, les cellules sont exposées aux nano-anticorps 3SM5 (A), 4N1H (B), 238D2 (C) et ovalbumine (A, B, C) à 100 nM pendant 1 heure à 4°C. La liaison des nano-anticorps est révélée à l'aide d'un anticorps anti-His couplé à l'allophycocyanine (anti-His-APC). La mesure est réalisée à l'aide du MACSQuant Analyzer 10 (Miltenyi Biotec).

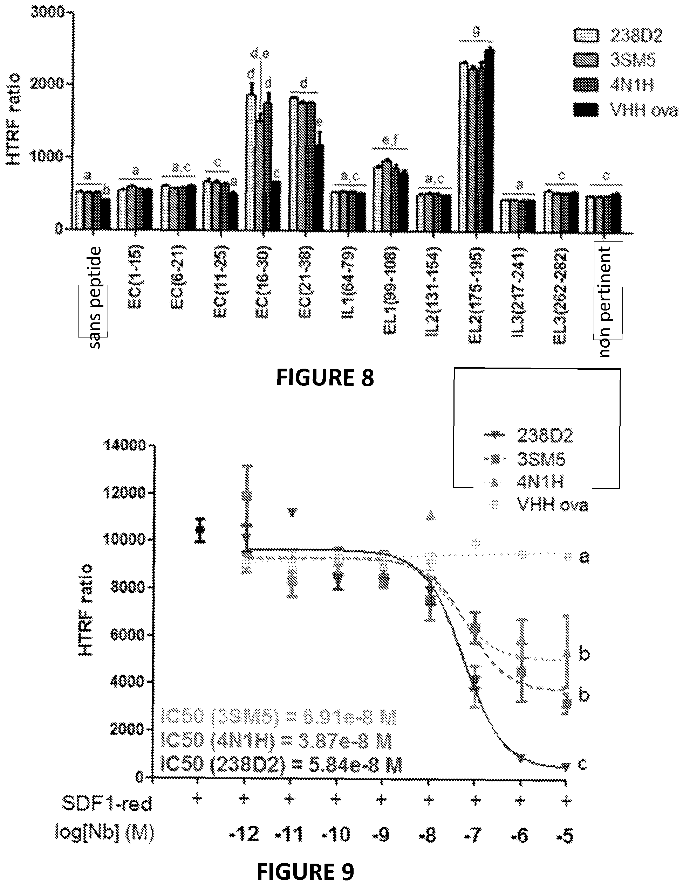

**Figure 8** : 3SM5 et 4N1H lie le même épitope sur le CXCR4 que le 238D2. Des peptides correspondant aux segments indiqués du CXCR4 ont été synthétisés et liés à une biotine en N-terminal. Chaque peptide (à 2 mM) a été mis en présence avec 2 µM de nano-anticorps couplé au tag His, avec 50 nM d'anticorps anti-His couplé à un donneur d'énergie, le terbium, et avec 2,35 µM de streptavidine couplée à l'accepteur d'énergie d2. Le transfert d'énergie entre le Tb et le d2 est mesuré à l'aide du Mithras² LB 943 Monochromator Multimode Reader (Berthold Technologies).
**Figure 9** : 3SM5 et 4N1H déplacent le ligand SDF1 du CXCR4, comme le 238D2. Des cellules HEK293 ont été transitoirement transfectées avec le CXCR4 fusionné au tag SNAP (Cisbio Bioassays). Quarante-huit heures après la transfection, les cellules sont traitées pour insérer le donneur terbium sur le tag SNAP. Le ligand SDF1 couplé à l'accepteur d2 est ajouté à 12 nM. Les cellules sont ensuite incubées avec les nano-anticorps 3SM5, 4N1H, 238D2 et ovalbumine aux concentrations indiquées pendant 1 heure. Le transfert d'énergie est mesuré à l'aide du Mithras² LB 943 Monochromator Multimode Reader (Berthold Technologies).

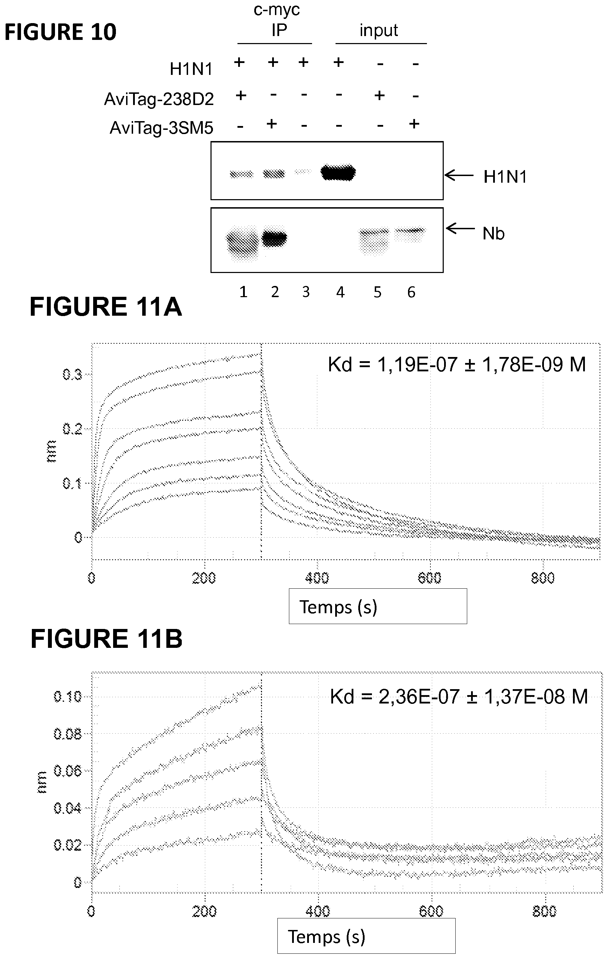

**Figure 10** : 238D2 immunoprécipite avec l'H1N1, comme le 3SMS. Des billes magnétiques ont été couplées avec un anticorps anti-myc et exposées à un mélange de H1N1 et de nano-anticorps couplé au tag myc et à l'AviTag biotinylé pendant 1 heure à 4°C. Les billes ont été lavées plusieurs fois et éluées. Les éluats ont été déposés en gel d'acrylamide puis transférés sur membrane de nitrocellulose. Les membranes ont été hybridées avec une streptavidine couplée à l'AlexaFluor680 pour révéler les nano-anticorps (panneau du bas) et à un anticorps anti-His pour révéler l'H1N1 (panneau du haut). Des aliquots des protéines purifiées n'ayant pas subit la procédure d'immunoprécipitation ont également été déposés dans le gel et apparaissent sous le nom d' «input».
**Figure 11** : 238D2 et 3SM5 lient l'H1N1 avec la même affinité. Des biosenseurs streptavidine d'interfermoterie sont chargés avec les nano-anticorps couplés à l'AviTag biotinylé, puis exposés à l'H1N1 à différentes concentrations allant de 84,75 à 847,5 nM. La dissociation a lieu dans le tampon de travail. Les constantes de dissociations (Kd) indiquées sur les graphiques ont été obtenus par le fit mathématique simultané de toutes les courbes.

### Description Détaillée de l'invention

La présente invention concerne une méthode permettant, à partir d'un anticorps de référence, d'identifier des anticorps de même spécificité (c'est-à-dire se liant à la cible de l'anticorps de référence) parmi une banque de données comprenant des caractérisations d'anticorps. De la même manière, la présente invention concerne également une méthode permettant, à partir d'un anticorps de référence, de déterminer d'autres cibles de cet anticorps.

Ainsi, la méthode selon la présente invention est adaptée :
- pour identifier de nouveaux anticorps spécifiques d'une cible donnée sur la base d'un anticorps de référence spécifique de celle-ci ; ou
- pour identifier de nouvelles cibles pour un anticorps de référence donné.

Dans la présente demande, « cible » d'un anticorps désigne une molécule biologique qui est spécifiquement liée par l'anticorps. Le terme « cible » peut également être remplacé par « antigène ».

Cette méthode est basée sur la mesure de la similitude de l'anticorps de référence avec les anticorps contenus dans une banque. Ainsi, la base de données comprend la caractérisation d'au moins 100, 150, 200, ou 1000 anticorps.

Dans cette base de données, les anticorps sont caractérisés par les propriétés de leurs CDRs (Complementary Determining Région ou Région Déterminante Complémentaire).

Notamment, la méthode est basée sur une analyse des CDRs permettant de quantifier des similitudes entre un anticorps de référence ou modèle et des anticorps de la base de données, notamment *via* le dénombrement des sous-séquences communes dans les CDRs.

De préférence, l'étape de prédiction *in silico* est suivie d'une étape de validation expérimentale, *in vitro.* Cette étape de validation expérimentale peut éventuellement comprendre des tests *in vivo* dans des modèles animaux.

La méthode mise au point par les inventeurs a été validée, comme cela est montré dans les exemples, en appliquant la méthode de la présente invention pour identifier des anticorps capables de cibler le récepteur CXCR4. En particulier, sur la base de deux anticorps connus pour se lier spécifiquement au récepteur CXCR4, les inventeurs ont abouti à l'identification de deux autres anticorps capables de se lier à CXCR4, bien que leurs cibles connues soient sans rapport avec CXCR4. Ces deux anticorps présentent un effet antagoniste de l'activité du récepteur, tout comme l'anticorps de référence utilisé pour les identifier. Enfin, les inventeurs ont également observé que l'anticorps de référence utilisé pour identifier les deux nouveaux anticorps est capable de se lier à la cible de l'un des nouveaux anticorps. Par ailleurs, il est intéressant de noter que ces anticorps n'auraient pas pu être identifiés par les méthodes classiques.

Ainsi, la méthode selon la présente invention est adaptée à l'identification, à partir d'un anticorps de référence, d'autres anticorps ayant la même spécificité et des propriétés pharmacologiques similaires à celles de l'anticorps de référence. Ainsi, lorsqu'un anticorps présente des propriétés pharmacologiques potentiellement intéressantes mais des propriétés physico-chimiques le rendant inutilisable (par exemple, toxicité, faible solubilité, difficulté de production), cette méthode peut permettre de trouver un nouveau candidat ayant les mêmes potentialités sans les inconvénients associés.

La méthode permet également pour un anticorps donné de déterminer des cibles possibles avec lesquelles cet anticorps est susceptible d'interagir. Dans ce contexte, cette méthode peut permettre d'anticiper les réactions croisées potentielles, sources éventuelles d'effets secondaires néfastes, en amont du développement d'un anticorps thérapeutique.

Cette méthode permet donc d'identifier très rapidement des anticorps de même spécificité qu'un anticorps de référence ainsi que des cibles alternatives pour cet anticorps de référence puisque la recherche se fait *in silico.* Cette identification est de préférence suivie d'une étape de validation par des expérimentations *in vitro.*

### Base de données selon la présente invention

La base de données selon la présente invention comprend des caractérisations pour chaque anticorps. En particulier, elle comprend, pour chaque anticorps :
- une séquence simplifiée des CDRs; et
- les structures secondaires des CDRs.

En particulier, la base de données selon la présente invention comprend, pour chaque domaine variable:
- Une séquence complète du domaine variable ;
- Une séquence simplifiée des CDRs du domaine variable ;
- Une structure secondaire des CDRs du domaine variable ; et
- Si disponible, la cible reconnue par l'anticorps comprenant le domaine variable, et en particulier l'épitope reconnu par l'anticorps.

Ainsi, comme détaillé ci-dessous, à chaque acide aminé des séquences des CDRs est assigné un ensemble d'attributs, ces attributs étant définis par la nature biochimique de l'acide aminé et par le type de structure secondaire auquel il contribue.

La base de données est construite sur la base des séquences des domaines variables des anticorps.

Les données des anticorps introduites dans la base de données correspondent à des nano-anticorps (nanobody).

Les nano-anticorps ou anticorps à domaine unique sont des anticorps naturels bien connus, consistant en des anticorps à chaîne lourde dépourvus de chaînes légères. Leur domaine variable est typiquement formé d'une pluralité de régions, dont une pluralité de régions charpentes conservées, dites FR, et une pluralité de régions hypervariables, déterminant la complémentarité avec l'antigène, dites CDRs.

Ainsi, si l'anticorps initial est un anticorps divalent comme une immunoglobine (IgG), les domaines variables de la chaîne lourde et de la chaîne légère sont considérés chacun comme des nano-anticorps et sont traités séparément. Si l'anticorps initial est un anticorps simple-chaîne (scFv), les deux parties variables sont considérées, et de même que pour une IgG, le domaine variable de la chaîne lourde et celui de la chaîne légère sont considérés chacun comme des nano-anticorps et sont traités séparément.

La séquence du domaine variable est traduite en un code simplifié. Ainsi, chaque acide aminé est remplacé par un code représentant une catégorie d'acides aminés, les catégories étant basées sur les caractéristiques physico-chimiques des acides aminés (par exemple, Bourquard et al., PLoS One, 2011, 6(4): e18541). Eventuellement, la séquence simplifiée peut être uniquement déterminée pour les séquences des CDRs des domaines variables, et non sur toute la séquence du domaine variable.

Le nombre de catégories utilisées pour recoder les séquences est inférieur à 15, de préférence à 10. Le nombre de catégories peut être compris entre 4 et 10, de préférence entre 5 et 9, de manière encore plus préférée est d'environ 6.

Les catégories basées sur les caractéristiques physico-chimiques des acides aminés sont ou sont choisies parmi la taille des acides aminés, leur hydrophobicité, leur neutralité, leur polarité, leur charge, leur caractère aromatique et des combinaisons de ces caractéristiques. Les catégories peuvent ainsi être une ou plusieurs catégories choisies parmi les suivantes : très petit, petit, aliphatique, aromatique, hydrophobe, polaire, chargé positif, chargé négatif, et des combinaisons de celles-ci.

Par exemple, dans un mode de réalisation particulier, les catégories utilisées sont les suivantes :
- « petit » comprenant les acides aminés suivants A, G, S, T, C et P et désigné par 5 ;
- « aromatique » comprenant les acides aminés suivants : Y, F et W et désigné par A ;
- « hydrophobe » comprenant les acides aminés suivants : I, L, F, M et V et désigné par H
- « polaire » comprenant les acides aminés suivants : N et Q et désigné par P ;
- « chargé positif » comprenant les acides aminés suivants : H, K et R et désigné par Q ;
- « chargé négatif » comprenant les acides aminés suivants : D et E et désigné par N.

Des exemples de simplification de séquences sont illustrés dans la présente demande.

La base de données comprend en outre la structure secondaire des CDRs.

La structure secondaire (2D) peut être déterminée par tout logiciel adéquat. Toute méthode permettant de déterminer la structure secondaire peut être utilisée. Il existe plusieurs méthodes pour définir la structure secondaire d'une protéine (par exemple STRIDE (Frishman D., Argos P., Proteins, vol. 23, n° 4, 1995, p. 566-579) ; ou DEFINE (Richards F. M., Kundrot C. E., Proteins, vol. 3, n° 2, 1988, p. 71-84). Une des méthodes préférées est la méthode du dictionnaire de structure secondaire de protéines (DSSP) (Touw et al. Nucleic Acids Research 2015; 43: D364-D368. ; Kabsch & Sander. Biopolymers. 1983, 22, 2577-2637). Ce dernier est couramment utilisé pour décrire la structure des protéines en utilisant des codes à une lettre.

Notamment, la structure secondaire est déterminée par un logiciel utilisant une structure tridimensionnelle (3D) du domaine variable.

La structure 3D du domaine variable peut être disponible dans une base de données, en particulier une base de données publique comme PDB (Protein Data Bank). Cette banque PDB est une collection mondiale de données sur la structure tridimensionnelle de macromolécules biologiques, dont les anticorps. Lorsque l'information est disponible, les caractéristiques de structure secondaire peuvent également être disponibles dans la base de données PDB.

Si la structure 3D n'est pas disponible, alors celle-ci peut être obtenue par modélisation par homologie, de préférence en prenant comme structure support l'anticorps dont la structure est connue et dont les CDRs sont les plus proches de ceux de l'anticorps à modéliser (mêmes longueurs et maximisant l'identité de séquence). Des logiciels sont disponibles pour réaliser cette modélisation, par exemple le logiciel Modeller (Webb et al, Current Protocols in Bioinformatics, John Wiley& Sons, Inc., 5.6.1-5.6.32, 2014 ; Marti-Renom et al. Annu. Rev. Biophys. Biomol. Struct. 29, 291-325, 2000 ; Sali & Blundell. J. Mol. Biol. 234, 779-815, 1993 ; Fiser et al. Protein Science 9. 1753-1773, 2000).

Ainsi, à chaque acide aminé des CDRs est associée une structure secondaire. Les catégories de structures secondaires peuvent être par exemple choisies parmi les suivantes :
- hélice 3₁₀ pouvant être désignée par exemple par G
- hélice α pouvant être désignée par exemple par H ;
- hélice π pouvant être désignée par exemple par I ;
- coude fermé pouvant être désigné par exemple par T ;
- brin β pouvant être désigné par exemple par E ;
- résidu dans un pont β pouvant être désigné par exemple par B ;
- coude pouvant être désigné par exemple par 5 ;
- une absence de structure secondaire.

De manière alternative, les catégories de structures secondaires peuvent également être codées par un chiffre.

Ainsi, chaque résidu des CDRs peut être associé à une catégorie de structure secondaire, pouvant éventuellement regroupée plusieurs catégories telles que décrites ci-dessus.

Ainsi, les CDRs peuvent également être traduits en une séquence où chaque acide aminé de la séquence initiale des CDRs est remplacé par un code représentant une catégorie de structure secondaire et le nombre de catégories étant compris entre 3 et 10, de préférence entre 4 et 9, de manière encore plus préférée entre 5 et 8.

Les CDRs des domaines variables peuvent être extraits par toute méthode disponible à l'homme du métier. Dans un mode de réalisation préférée, la méthode de Chlothia est mise en oeuvre pour déterminer les CDRs (Chothia et al, Nature 342, 877 - 883). Cependant, toute autre méthode alternative de détermination des CDRs peut être mise en oeuvre tant que la méthode utilisée pour la base de données est homogène dans celle-ci. Par exemple, et à titre non-limitatif, on peut citer la méthode de Kabat (Kabat et al., 1991, Séquences of Proteins of Immunological Interest, 5th Ed., United States Public Health Service, National Institutes of Health, Bethesda), la méthode intermédiaire entre Chlothia et Kabat appelé AbM (logiciel de modélisation d'anticorps AbM de Oxford Molecular) ou la méthode dite "Contact" basée sur une analyse de structures de complexes disponibles.

Enfin, la base de données comprend également, lorsque l'information est disponible, la cible de l'anticorps, et en particulier éventuellement l'épitope fixé par l'anticorps.

La présente invention est donc également relative à une telle banque de données comprenant une pluralité de domaines variables, avec pour chaque domaine variable, une séquence simplifiée des CDRs de chaque domaine variable et la structure secondaire des CDRs de chaque domaine variable. De préférence, la base de données comprend en outre la cible ou les cibles de l'anticorps comprenant le domaine variable. La base de données peut comprendre des informations complémentaires pour chaque domaine variable comme la séquence de l'épitope reconnu par l'anticorps, l'activité biologique de l'anticorps sur la cible (par exemple, agoniste, antagoniste, bloquant, inhibiteur, activateur, etc...), la structure tridimensionnelle de l'anticorps, la séquence simplifiée du domaine variable, le modèle de la structure tridimensionnelle s'il a été nécessaire de le réaliser, et dans ce cas la structure support utilisée pour la modélisation.

De préférence, la base de données comprend au minimum 100, 150, 200, 250, ou 300 anticorps. Dans un mode de réalisation préféré, la base de données comprend au minimum 2 anticorps différents par cible, de préférence au minimum 3 anticorps. Dans un mode de réalisation préféré, la base de données comprend tous les anticorps dont la séquence protéique est connue et pour lesquels soit la structure tridimensionnelle est disponible, soit un modèle par homologie de celle-ci a pu être réalisé.

### Similitude

Sur la base des séquences simplifiées des CDRs et des structures secondaires des CDRs de la banque de données, la méthode comprend un calcul de similitude entre un anticorps de référence et des anticorps de la base de données selon la présente invention.

Il existe un grand nombre de méthodes disponibles pour calculer des similitudes.

Dans un mode de réalisation préféré, la méthode est basée sur le dénombrement de sous-séquences communes dans les CDRs de deux anticorps, permettant ainsi de quantifier la similitude de l'anticorps de référence et d'un autre anticorps test de la base de données. Cette similitude est mesurée par un score qui correspond à une distance entre deux anticorps et est basée sur le dénombrement de sous-séquences. La méthode de calcul de similitude a été développée sur la base de l'enseignement d'Egho et al (Research Report, RR-8086, 2012, 1-19).

Une distance faible (c'est-à-dire une grande similitude) est indicative d'une capacité de liaison à la même cible que l'anticorps de référence. Un indice supplémentaire à prendre en compte est le nombre d'anticorps de référence ayant la même cible et qui présentent une similitude élevée avec un même anticorps test. Si plusieurs anticorps de référence présentent une similitude importante, alors la valeur de distance peut être un peu plus élevée que lorsqu'un seul anticorps de référence présente une similitude.

Des valeurs numériques de similitude de référence ou seuil peuvent être facilement définies par l'utilisateur pour définir la valeur numérique de similitude de référence ou seuil utile pour prédire que l'anticorps test et l'anticorps de référence sont capables de se lier à la même cible. Par exemple, ces valeurs pourront être déterminées avec des anticorps connus pour fixer la même cible et facultativement avec des anticorps connus pour ne pas se fixer aux mêmes cibles.

Ainsi, les anticorps étant à une distance d'une valeur inférieure ou égale à 4 par rapport à un anticorps de référence sont prédits être capables de se lier à la même cible que l'anticorps de référence.

De manière complémentaire, les anticorps étant à une distance d'une valeur comprise entre 4 et 6 par rapport à plusieurs anticorps de référence présentant la même cible sont prédits être capables de se lier à la même cible que les anticorps de référence.

Les anticorps correspondant à une valeur prédisant une similitude de cible sont sélectionnés. Ces anticorps sélectionnés sont désignés par l'appellation anticorps similaire. Ces anticorps peuvent ensuite faire l'objet d'une validation expérimentale pour tester leur spécificité pour la cible.

### Validation expérimentale

De préférence, l'identification *in silico* d'un anticorps potentiel capable de se lier à la cible, ou anticorps similaire, est validée expérimentalement. Ainsi, cette étape de validation peut permettre de confirmer la capacité d'un anticorps similaire à se lier à la cible de l'anticorps de référence ou de confirmer la capacité d'un anticorps de référence à se lier à la cible de l'anticorps similaire.

La méthode selon la présente invention peut donc comprendre une étape supplémentaire de validation *in vitro* de la capacité de liaison de l'anticorps similaire ou l'anticorps de référence à la cible de l'anticorps de référence ou la cible de l'anticorps similaire, respectivement.

Ainsi, dans un premier mode de réalisation, l'étape de validation est une étape de validation *in vitro* de la capacité de liaison de l'anticorps similaire à la cible de l'anticorps de référence.

La méthode peut comprendre la fourniture ou la production de l'anticorps identifié ou anticorps similaire, sa mise en contact avec la cible de l'anticorps de référence, et la mesure de la liaison entre l'anticorps identifié ou similaire et la cible.

De préférence, l'anticorps identifié ou anticorps similaire peut être fourni sous sa forme originale ou les CDRs (en particulier CDR1, CDR2 et CDR3) de cet anticorps peuvent être greffés dans un squelette approprié. Dans un mode de réalisation préféré, le squelette préféré sera celui d'un nano-anticorps, encore appelé VHH. De manière alternative, le squelette dans lequel les CDRs peuvent être greffés est celui de la chaîne lourde VH d'anticorps. Dans ce contexte, la chaîne lourde sera ou non associée à une chaîne légère.

Dans un second mode de réalisation, l'étape de validation est une étape de validation *in vitro* de la capacité de liaison de l'anticorps de référence à la cible de l'anticorps similaire.

La méthode peut comprendre la production ou la fourniture de l'anticorps de référence, sa mise en contact avec la cible de l'anticorps similaire, et la mesure de la liaison entre l'anticorps de référence et la cible de l'anticorps similaire.

L'anticorps de référence peut être fourni sous sa forme originale ou les CDRs (en particulier CDR1, CDR2 et CDR3) de cet anticorps peuvent être greffés dans un squelette approprié. Dans un mode de réalisation préféré, le squelette préféré sera celui d'un nano-anticorps, encore appelé VHH. De manière alternative, le squelette dans lequel les CDRs peuvent être greffés est celui de la chaîne lourde VH d'anticorps. Dans ce contexte, la chaîne lourde sera ou non associée à une chaîne légère.

Dans un mode de réalisation préféré, le squelette d'un nano-anticorps, encore appelé VHH, sera préféré pour l'étape de validation *in vitro.* Ainsi, les CDRs (CDR1, CDR2 et CDR3) sont greffés dans un squelette de nano-anticorps comprenant les régions charpentes FR1, FR2, FR3 et FR4 pour former un nano-anticorps comprenant les segments FR1-CDR1-FR2-CDR2-FR3-CDR3-FR4 (une première région charpente FR1, une première région hypervariable CDR1, une deuxième région charpente FR2, une deuxième région hypervariable CDR2, une troisième région charpente FR3, une troisième région hypervariable CDR3, et une quatrième région charpente FR4).

FR1, FR2, FR3 et FR4 peuvent exister dans des régions de VHH de *Camelidae,* de préférence de dromadaire, de chameau, de lama ou d'alpaca. FR1, FR2, FR3 and FR4 peuvent également être de régions humanisées de VHH. Par exemple, la demande WO2015/063331 illustre la préparation d'anticorps à domaine unique synthétiques.

De nombreux squelettes de nano-anticorps sont maintenant disponibles. Par exemple, la première région charpente pourrait être choisie parmi MEVQLQESGGGLVQAGASLKLSCAAS (SEQ ID No 1) ou MEVQLVQSGAEVKKPGASVKVSCKAS (SEQ ID No 2), la deuxième région charpente pourrait être choisie parmi MGWFRQAPGKEREFVAA (SEQ ID No 3) ou HINWVRQAPGQGLEWMGW (SEQ ID No 4), la troisième région charpente pourrait être choisie parmi TKYADSVKGRFAISRDNDKNTVWLRMNSLKPEDTAVYYC (SEQ ID No 5) ou NYAQKFQGWVTMTRDTAISTAYMEVNGLKSDDTAVYYC (SEQ ID No 6), et la quatrième région charpente être WGQGTQVTVSS (SEQ ID No 7), ou toutes séquences présentant au moins 80, 85, 90 ou 95 % d'identité avec celles-ci. Dans un mode de réalisation particulier, la première région charpente pourrait être MEVQLQESGGGLVQAGASLKLSCAAS (SEQ ID No 1), la deuxième région charpente MGWFRQAPGKEREFVAA (SEQ ID No 3), la troisième région charpente TKYADSVKGRFAISRDNDKNTVWLRMNSLKPEDTAVYYC (SEQ ID No 5), et la quatrième région charpente WGQGTQVTVSS (SEQ ID No 7), ou toutes séquences présentant au moins 80, 85, 90 ou 95 % d'identité avec celles-ci. Dans un deuxième mode de réalisation particulier, la première région charpente pourrait être MEVQLVQSGAEVKKPGASVKVSCKAS (SEQ ID No 2), la deuxième région charpente HINWVRQAPGQGLEWMGW (SEQ ID No 4), la troisième région charpente NYAQKFQGWVTMTRDTAISTAYMEVNGLKSDDTAVYYC (SEQ ID No 6), et la quatrième région charpente WGQGTQVTVSS (SEQ ID No 7), ou toutes séquences présentant au moins 80, 85, 90 ou 95 % d'identité avec celles-ci.

Cette étape de validation peut être réalisée par toute méthode disponible à l'homme du métier. De manière non-exhaustive, on peut citer par exemple par des tests ELISA, des tests en interférométrie ou en résonnance plasmonique de surface, ou par une quelconque méthode impliquant le transfert d'énergie entre molécules fluorescentes.

La mise en oeuvre de la méthode est illustrée dans les exemples suivants.

### Exemples

### Exemple 1 - Identification de nouveaux anticorps ciblant le récepteur CXCR4 et identification d'une cible alternative pour l'anticorps de référence.

### Résumé

La preuve de concept de la méthode a été réalisée en prenant comme anticorps de référence un anticorps ciblant le récepteur CXCR4 (238D2). Cet anticorps a été décrit dans la publication Jähnichen et al. (2010, PNAS, 107, 20565-70 ; US 2011/0117113 A1).

Deux anticorps homologues ont été identifiés par la méthode selon la présente invention : un VHH de Camélidé ciblant la β-lactamase (code PDB 4N1H) et un domaine VH d'un anticorps humain ciblant l'hémagglutinine du virus H1N1 (strain A/Solomon Islands/3/2006, code PDB 3SM5).

Aucun de ces deux anticorps n'avait été testé pour sa capacité à reconnaître le récepteur CXCR4, et il n'existe aucune ressemblance globale, ni en séquence, ni en structure entre CXCR4 et la β-lactamase d'une part ou entre CXCR4 et l'hémagglutinine d'autre part.

Par des expériences de cytométrie, les inventeurs ont montré que 3SM5 et 4N1H reconnaissent le récepteur CXCR4 humain exprimé transitoirement en cellules HEK293. Des études de FRET et de BRET avec ces mêmes cellules ont permis de montrer que les deux anticorps 3SM5 et 4N1H inhibent l'activité du récepteur CXCR4, comme le fait l'anticorps de référence.

Les inventeurs ont pu démontrer expérimentalement que, comme l'anticorps de référence, les deux anticorps identifiés *in silico* par la méthode selon l'invention inhibent l'activation de la voie de signalisation protéines G-dépendante ainsi que le recrutement des β-arrestines par le récepteur CXCR4 activé par son ligand naturel, la chémokine SDF-1α. L'activité antagoniste des anticorps identifiés s'exerce à partir de concentrations de l'ordre du nM et est dépendante de la dose d'anticorps. Un VHH dirigé contre l'ovalbumine a été utilisé comme contrôle négatif, si il présente une faible activité à des doses de l'ordre du nM, cette activité n'est pas dose-dépendante, et donc non spécifique.

### Résultats

La méthode a été appliquée en prenant comme anticorps initiaux les domaines variables des VHH « 238D2 » et « 238D4 » reconnaissant le récepteur CXCR4 humain.

Les structures 3D n'étant pas disponibles, des modèles 3D ont été réalisés en utilisant comme support la structure PDB 3SN6 pour les deux anticorps.

Les inventeurs ont ainsi pu identifier deux anticorps candidats :
- Le domaine VH de l'anticorps CH65, qui cible l'hemagglutinine du virus influenza souche H1N1 (strain A/Solomon Islands/3/2006), code PDB 3SM5 (Whittle, et al. (2011). Proceedings of the National Academy of Sciences, 108(34), 14216-14221). Ce nano-anticorps présente une similitude de 3,6 avec le nano-anticorps initial 238D2.
- Le domaine variable de l'anticorps mono-chaîne cAB-F11N, qui cible la β-lactamase de la bactérie *Bacillus licheniformis,* code PDB 4N1H (non publié). Ce nano-anticorps présente une similitude de 4,2 avec le nano-anticorps 238D4 et de 6 avec le nano-anticorps 238D2.

Les domaines variables de cAB-F11N (ci-après nommé 4N1H) et de la chaîne lourde de CH65 (ci-après nommé 3SM5) ont été produits en utilisant la séquence reportée dans la PDB, en ajoutant un tag myc et un tag his.

### 4N1H:

SEQ ID NO 8 : 
MEVQLQESGGGLVQAGASLKLSCAASGRTFSSYAMGWFRQAPGKEREFVAAISRSGGDTKYADSVKGRFAISR
DNDKNTVWLRMNSLKPEDTAVYYCAATTYASLSDTYIGEHIYDDWGQGTQVTVSS
SEQ ID NO 9 (4N1H - tag myc) : 
MEVQLQESGGGLVQAGASLKLSCAASGRTFSSYAMGWFRQAPGKEREFVAAISRSGGDTKYADSVKGRFAISR
DNDKNTVWLRMNSLKPEDTAVYYCAATTYASLSDTYIGEHIYDDWGQGTQVTVSSEQKLISEEDLE
SEQ ID NO 10 (4N1H - tag his) :

MEVQLQESGGGLVQAGASLKLSCAASGRTFSSYAMGWFRQAPGKEREFVAAISRSGGDTKYADSVKGRFAISR
DNDKNTVWLRMNSLKPEDTAVYYCAATTYASLSDTYIGEHIYDDWGQGTQVTVSSEQKLISEEDLEHHHHHH

### 3SM5

SEQ ID NO 11 : 
MEVQLVQSGAEVKKPGASVKVSCKASGYTFTDYHINWVRQAPGQGLEWMGWIHPNSGDTNYAQKFQGWV
TMTRDTAISTAYMEVNGLKSDDTAVYYCARGGLEPRSVDYYYYGMDVWGQGTTVTVSS
SEQ ID NO 12 (3SM5 - tag myc) : 
MEVQLVQSGAEVKKPGASVKVSCKASGYTFTDYHINWVRQAPGQGLEWMGWIHPNSGDTNYAQKFQGWV
TMTRDTAISTAYMEVNGLKSDDTAVYYCARGGLEPRSVDYYYYGMDVWGQGTTVTVSSEQKLISEEDLE
SEQ ID NO 13 (3SM5 - tag his) : 
MEVQLVQSGAEVKKPGASVKVSCKASGYTFTDYHINWVRQAPGQGLEWMGWIHPNSGDTNYAQKFQGWV
TMTRDTAISTAYMEVNGLKSDDTAVYYCARGGLEPRSVDYYYYGMDVWGQGTTVTVSSEQKLISEEDLEHHH
HHH
SEQ ID NO 38 (AviTag - 3SM5 - tag myc)

MGSSHHHHHHSSGLVPRGSHMSGLNDIFEAQKIEWHEDPNSEVQLVQSGAEVKKPGASVKVSCKASGYTFTD
YHINWVRQAPGQGLEWMGWIHPNSGDTNYAQKFQGWVTMTRDTAISTAYMEVNGLKSDDTAVYYCARGG
LEPRSVDYYYYGMDVWGQGTTVTVSSEQKLISEEDL

### 238D2

SEQ ID NO 39 (238D2- tag myc) 
MEVQLVESGGGLVQTGGSLRLSCAASGFTFSSYAMSWVRQAPGKGLEWVSGIKSSGDSTRYAGSVKGRFTISR
DNAKNMLYLQMYSLKPEDTAVYYCAKSRVSRTGLYTYDNRGQGTQVTVSSEQKLISEEDLE
SEQ ID NO 40 (238D2- tag his) 
MEVQLVESGGGLVQTGGSLRLSCAASGFTFSSYAMSWVRQAPGKGLEWVSGIKSSGDSTRYAGSVKGRFTISR
DNAKNMLYLQMYSLKPEDTAVYYCAKSRVSRTGLYTYDNRGQGTQVTVSSEQKLISEEDLEHHHHHH
SEQ ID NO 41 (AviTag - 238D2 - tag myc)

MGSSHHHHHHSSGLVPRGSHMSGLNDIFEAQKIEWHEDPNSEVQLVESGGGLVQTGGSLRLSCAASGFTFSSY
AMSWVRQAPGKGLEWVSGIKSSGDSTRYAGSVKGRFTISRDNAKNMLYLQMYSLKPEDTAVYYCAKSRVSRT
GLYTYDNRGQGTQVTVSSEQKLISEEDL

### 238D4

SEQ ID NO 42 (238D4- tag myc) 
MEVQLMESGGGLVQAGGSLRLSCAASGRTFNNYAMGWFRRAPGKEREFVAAITRSGVRSGVSAIYGDSVKDR
FTISRDNAKNTLYLQMNSLKPEDTAVYTCAASAIGSGALRRFEYDYSGQGTQVTVSSEQKLISEEDLE
SEQ ID NO 43 (238D4- tag his)

MEVQLMESGGGLVQAGGSLRLSCAASGRTFNNYAMGWFRRAPGKEREFVAAITRSGVRSGVSAIYGDSVKDR
FTISRDNAKNTLYLQMNSLKPEDTAVYTCAASAIGSGALRRFEYDYSGQGTQVTVSSEQKLISEEDLEHHHHHH

### Ovalbumine

SEQ ID NO 44 (Ovalbumine - tag his) 
MGSSHHHHHHSSGLVPRGSHMASMTGGQQMGRGSEFELRRQACGRTRAPPPPPLRSGCEVQLQESGGSGQ
AGGSLRLSCAASGDTVRTMAWFRQAPGQEREGVAGFNLPISRPYYADGMKARFTISGDKSKNTVTLQMDNLA
PEDTANYYCAATRYTLDLSSRIFQGDFDHWGHGTQVTVSS
**SEQ ID NO 45 (CXCR4)**

MEGISIYTSDNYTEEMGSGDYDSMKEPCFREENANFNKIFLPTIYSIIFLTGIVGNGLVILVMGYQKKLRSMTDKY
RLHLSVADLLFVITLPFWAVDAVANWYFGNFLCKAVHVIYTVNLYSSVWILAFISLDRYLAIVHATNSQRPRKLLA
EKVVYVGVWIPALLLTIPDFIFANVSEADDRYICDRFYPNDLWVVVFQFQHIMVGLILPGIVILSCYCIIISKLSHSGS
NIFEMLRIDEGLRLKIYKDTEGYYTIGIGHLLTKSPSLNAAKSELDKAIGRNTNGVITKDEAEKLFNQDVDAAVRGIL
RNAKLKPVYDSLDAVRRAALINMVFQMGETGVAGFTNSLRMLQQKRWDEAAVNLAKSRWYNQTPNRAKRV
ITTFRTGTWDAYGSKGHQKRKALKTTVILILAFFACWLPYYIGISIDSFILLEIIKQGCEFENTVHKWISITEALAFFHC
CLNPILYAFLGAKFKTSAQHALTSVSRGSSGRPLEVLFQ

Les anticorps ont été produits dans E coli.

Différents tests fonctionnels ont été réalisés ensuite pour démontrer l'effet fonctionnel et la spécificité des deux nouveaux anticorps identifiés.
- L'effet sur le recrutement des ß-arrestines ligand-dépendant (Figure 3). Des cellules exprimant le récepteur sont exposées à une dose fixe de SDF-1, agoniste naturel du récepteur CXCR4, et à des doses croissantes d'anticorps. Le recrutement des β-arrestines est mesuré par le signal BRET entre la queue C-terminale du récepteur et la β-arrestine. On a observé une inhibition dose-dépendante du recrutement des β-arrestines par les deux nouveaux anticorps, inhibition semblable à celle observée avec les deux anticorps initiaux, avec des IC50 comparables. Un VHH ciblant l'ovalbumine est utilisé comme contrôle négatif. Aucun effet de cet anticorps n'est observé sur l'effet du SDF-1 sur son récepteur.
- L'effet sur la production de second messager (Figure 4). Des cellules exprimant le récepteur sont exposées à une dose fixe de forskoline, qui a pour effet d'augmenter fortement la quantité d'AMPc dans la cellule, puis une dose fixe de SDF-1, qui par l'activation de la protéine Gi diminue fortement cette quantité d'AMPc, et à des doses croissantes d'anticorps. La quantité d'AMPc dans la cellule est mesurée par l'utilisation du senseur CAMyEL. On a observé l'inhibition de la diminution de la quantité d'AMPc dans la cellule par les quatre anticorps, ce qui démontre l'inhibition du récepteur. Les IC50 calculés sont comparables pour les quatre anticorps. Aucun effet n'est observé avec le VHH ovalbumine.

Les inventeurs ont également pu montrer par ELISA la liaison des anticorps 238D2, 238D4 et 4N1H à l'hémagglutinine (H1N1), la cible de l'anticorps nouvellement identifié comme étant spécifique de CXCR4, à savoir 3SM5 (Figure 5).

Comme le montrent les Figures 6 et 7, 3SM5 et 4N1H lient le CXCR4 surexprimé à la surface des HEK293, comme le 238D2 et 238D4. Aucune liaison (ou une liaison très faible) n'est observée avec le VHH ovalbumine.

Caractérisation de l'épitope (Figure 8) : Des peptides correspondant aux segments du CXCR4 et un peptide non relevant ont été synthétisés et couplés à une biotine en N-terminal. Ces peptides correspondent aux segments suivants, la séquence du CXCR4 étant décrites dans la SEQ ID NO 45 (Uniprot ID P61073-1) :
- dans le domaine extracellulaire du récepteur (EC) : aa 1-15, aa 6-21, aa 11-25, aa 16-30, aa 21-38
- boucle intracellulaire 1 (IL1) : aa 64-79
- boucle extracellulaire 1 (EL1) : aa 99-108
- boucle intracellulaire 2 (IL2) : aa 131-154
- boucle extracellulaire 2 (EL2) : aa 175-195
- boucle intracellulaire 3 (IL3) : aa 217-241
- boucle extracellulaire 3 (EL3) : aa 262-282

Ces peptides ont été mis en présence des nano-anticorps 238D2, 3SM5, 4N1H et ovalbumine tous couplés à un tag His. La liaison entre les peptides et les nano-anticorps a été mesurée par HTFR (transfert d'énergie) entre une streptavidine couplée à un donneur (qui lie la biotine du peptide) et un anticorps anti-His couplé à l'accepteur (qui se lie aux nano-anticorps). On a observé que les zones EC 16-30, EC 21-38, EL1 et EL2 lient les nano-anticorps 3SM5, 4N1H et 238D2 montrant que les épitopes des trois anticorps sont très similaires. Le nano-anticorps ovalbumine se lie également à la zone EL2 et à plus faible mesure à la zone EC 21-38, mais pas à la zone EC 16-30.
- L'effet sur la liaison du ligand SDF1 (Figure 9) : Des cellules exprimant le CXCR4 couplé à un tag SNAP sur lequel est lié un donneur d'énergie (ion terbium) ont été exposées au SDF1 couplé à un accepteur d2. Un signal FRET a été observé, qui indique que le SDF1 lie le CXCR4. Les cellules ont ensuite été exposées à des doses croissantes des nano-anticorps 238D2, 3SM5, 4N1H et ovalbumine. On a observé une diminution du signal FRET entre le SDF1 et le CXCR4 indiquant que les nano-anticorps 238D2, 3SM5 et 4N1H entrent en compétition avec le SDF1. Les IC50 sont de même ordre, indiquant une affinité similaire entre les 3 nano-anticorps. Le nano-anticorps ovalbumine n'a pas déplacé le ligand.
- Co-immunoprécipitation de H1N1 et 238D2 et 3SM5 (Figure 10) : Les nano-anticorps couplés à un tag myc ont été mélangés à l'H1N1. Le mélange a été mis en présence de billes magnétiques couplées à un anticorps anti-myc. On a observé que dans les conditions où le 238D2 et le 3SM5 sont présents, I'H1N1 précipite sur les billes, ce qui n'est pas le cas quand les nano-anticorps sont absents. L'H1N1 et le nano-anticorps 238D2 forment donc un complexe.
- Mesure d'affinité entre 238D2 et H1N1 ou 3SM5 et H1N1 (Figure 11) : Des biosenseurs couplés à la streptavidine ont été chargés avec les nano-anticorps 238D2 et 3SM5 couplés à l'AviTag biotinylé. Différentes doses d'H1N1 ont ensuite été mises en présence des biosenseurs, chacune induisant une courbe d'association. La dissociation a été réalisée de manière passive dans le tampon de travail. Les courbes sont fittées mathématiquement toutes en même temps pour donner la valeur de Kd (constante de dissociation) indiquée sur les graphiques. Le 238D2 et le 3SM5 lient l'H1N1 avec la même affinité.

### Matériaux & Méthodes

### Production et purification des anticorps.

Des E.coli sont transformées avec des vecteurs pET28b+ codant pour les VHH. Les bactéries sont induites à l'IPTG 1 mM, 4 heures à 37°C. Les lysats bactériens sont purifiés sur colonnes Ni-NDA (Macherey-Nagel, Düren, Germany) puis les élutions sont dialysées en Tris pH8 100 mM, NaCl 500 mM, glycérol 5%, à 4°C.

### Essais BRET pour la détection de la production d'AMPc et le recrutement de la β-arrestine 2 au récepteur

Le récepteur CXCR4 a été produit à partir du cDNA (Resource Center, University of Missouri-Rolla, USA), les constructions CXCR4-Rluc8, YPET-β arrestine 2, et CAMYEL ont été obtenues auprès du Dr. Mohammed Akli Ayoub. Des cellules HEK293 FT ont été transfectées dans des boites 96 puits avec 50 ng de plasmide et 0,5 µl de Métafectène (Biontex Laboratories GmbH, München, Germany) par puits, en accord avec les instructions du fournisseur. Quarante-huit heures après la transfection, les nano-anticorps ont été ajoutés aux cellules dans du PBS-Hepes 10 mM et laissés 2h à 37°C. Le SDF1-α (Peprotech, Rocky Hill, NJ, USA) a été ajouté à la concentration finale de 50 nM pendant 15 minutes. De la Coelentérazine H (Interchim, Montluçon, France) à la concentration finale de 50 µM a été ajoutée dans le milieu juste avant la mesure, ainsi que de la forskoline à la concentration finale de 100 µM pour l'essai CAMYEL puisque le récepteur CXCR4 est couplé à la protéine Gi. Les plaques ont été lues avec un Mithras² LB 943 Monochromator Multimode Reader (Berthold Technologies, Thoiry, France).

### ELISA H1N1

La protéine H1N1 (Sino Biological Inc, Beijing, P.R.China) a été déposée sur des plaques Maxisorp à 1 µg/ml, puis laissée une nuit à 4°C. Après 3 lavages au PBS 0,01% Tween 20, les plaques ont été saturées au PBS à 1% de lait. Les nano-anticorps ont été ajoutés pendant 2h dans le PBS - 1% lait aux concentrations indiquées. Les plaques ont été lavées 3 fois 5 minutes dans le PBS-Tween. L'anticorps couplé-HRP dirigé contre le tag myc (Abcam, Cambridge, UK) a été ajouté à la dilution 1:000 dans le PBS - 1% lait et incubé 1h à température ambiante. Les plaques ont été lavées 3 fois 5 minutes dans le PBS-Tween et 3 fois 5 minutes dans le PBS. Le substrat HRP (SuperSignal^{™} ELISA Pico Chemiluminescent Substrate, Thermo Fischer Scientific, MA, USA) dilué au 1:20 dans le PBS a été chargé dans les plaques et les mesures ont été réalisées avec le Mithras² LB 943 Monochromator Multimode Reader

### FACS

Les cellules ont été transitoirement transfectées avec le CXCR4 humain. Quarante-huit heures après transfection, les cellules ont été saturées une heure en PBS-BSA 1%, et exposées aux VHH dilués à 1µM en PBS-BSA pour la figure 6 et à 100 nM pour la figure 7.

Pour la Figure 6, après 2 lavages, les cellules ont été exposées à l'anticorps de révélation anti-His couplé à l'allophycocyanin (APC) en PBS-BSA 30 min à température ambiante. Le signal fluorescent a été enregistré au FACScalibur (BD Biosciences, San Jose, CA, USA).

Pour la Figure 7, après 2 lavages, les cellules ont été exposées à l'anticorps de révélation anti-His couplé à l'allophycocyanin (APC) en PBS-BSA 60 min à 4°C. Le signal fluorescent a été enregistré au MACSQuant Analyzer 10 (Miltenyi Biotech GmbH, Bergisch Gladbach, Germany).

Dix mille événements au moins ont été mesurés et les pics comparés au signal de l'anti-His-APC seul.

### Déplacement SDF1

Les cellules HEK293 ont été transitoirement transfectées avec le CXCR4 couplé à un tag SNAP (Cisbio Bioassays, France). Quarante-huit heures après la transfection les cellules ont été couplées à l'ion terbium selon le protocole du kit SNAP Lumi-4 terbium (Cisbio Bioassay). Après plusieurs lavage en tampon tag-lite labelling medium (Cisbio Bioassays), 20000 cellules ont été distribuées en plaque 384 puits. Les nano-anticorps sont ajoutées aux doses indiquées durant 1 heure à 37°C. Le SDF1 couplé à l'accepteur d2 a été ajouté à la concentration finale de 12 nM pendant. Après une nuit d'incubation à 4°C, les plaques ont été lues au Mithras² LB 943 Monochromator Multimode Reader (Berthold Technologies, Thoiry, France).

### Cartographie de l'épitope

Les peptides biotinylés en N-ter ont été synthétisés par Genecust. En plaque 384 puits, les peptides ont été déposés à la concentration finale de 2 mM avec les nano-anticorps à la concentration finale de 2 µM en PBS-0,1% tween 20. Après une nuit d'incubation à 4°C, 2,35 µM streptavidine couplée à l'accepteur d2, et 50 nM d'anticorps anti-His couplé au donneur Terbium ont été ajoutés. La plaque a été lue 24h plus tard, au Mithras² LB 943 Monochromator Multimode Reader (Berthold Technologies, Thoiry, France). Les résultats indiqués correspondent au ratio entre le signal émis par l'accepteur et le signal émis par le donneur.

### Interférométrie Biolaver (BLI)

Les mesures ont toutes été réalisées avec l' Octet RED96 System (Pall forte Bio, Fremont, CA, USA), dans le tampon kinetic buffer de Pall Forte, à 30°C et sous 1000 rpm d'agitation. Les nano-anticorps 238D2 et 3SM5 couplés à l'AviTag biotinylés ont été immobilisés sur des biosensuers streptavidin à 100 nM puis laissés en stabilisation 120 secondes dans le tampon. La liaison de l'Influenza A H1N1 (A/SOLOMON ISLANDS/3/2006) a été mesurée à 50, 30, 20, 15, 10, 7.5 and 5 µg/m! pendant 5 minutes. Une correction a été effectuée en soustrayant la liaison de I'H1N1 à une protéine biotinylée non pertinente. Les résultats ont été analysés avec le logiciel Octet Software 9.0 version, selon le modèle d'interaction 1:1. Une analyse globale de toutes les courbes ayant pour postulat que la dissociation est réversible a été réalisée. Un seul jeu de paramètres de liaison en résulte pour toutes les concentrations utilisées.

### Immunoprécipitation

Dix µg de nano-anticorps couplés à l'AviTag biotinylé et 10 µg d'Influenza A H1N1 (A/SOLOMON ISLANDS/3/2006) ont été mélangés dans un volume final de 500 µl de tampon TNET (20 mM Tris HCl pH 8, 137 mM NaCl, 1% Nonidet P-40, 2 mM EDTA) et laissés sur la nuit à 40°C sous agitation. Cinq cent microlitres de billes magnétiques couplées aux protéines A et G (Merck Millipore, armstadt, Germany) ont été équilibrés en tampon TNET avant d'être chargées avec 10 µl d'anticorps anti-myc 9E10 antibody (Abcam, Cambridge, MA, USA) sur la nuit à 4°C. Après 3 lavages en TNET, les billes ont été exposées au mélange nano-anticorps - H1N1 pendant 2h à 4°C. Les billes ont été lavées 3 fois en TNET et suspendues en bleu de charge 2X (0.02 % bromophenol blue, 125 mM Tris HCl pH6.8, 20 % Glycerol, 4 % SDS, 10 % β-ME). Les surnageant protéiques ont été chauffés à 100°C pendant 5 min, séparés sur SDS-PAGE et transférés sur membrane de nitrocellulose. Les membranes ont été saturées en Tris Buffer Saline - 0.01% Tween 20 (TBS-T) - 3% BSA pendant 30 min à température ambiante. Les membranes ont été hybridées avec un anticorps anti-His (Anti-6X His tag antibody - ChIP Grade, Abcam, Cambridge, MA, USA) lui-même révélé avec l'anticorps IRDye 680 secondaire de chèvre anti-lapin (Goat anti-Rabbit secondary antibody LI-COR, Lincoln, NE, USA). Simultanément, les membranes ont été hybridées avec une streptavidine couplée à l'AlexaFluor. Les membranes ont été scanées avec l'Odyssey CLx (LI-COR, Lincoln, NE, USA).

## Revendications

1. Méthode mise en oeuvre par ordinateur permettant d'identifier des anticorps capables de se lier à la même cible, la méthode comprenant :
- la fourniture d'une base de données comprenant la caractérisation d'au moins 100 anticorps, chaque anticorps étant **caractérisé par** une séquence simplifiée et une structure secondaire des CDRs (Région Déterminante Complémentaire) CDR1, CDR2 et CDR3 pour chaque domaine variable d'un anticorps de la base de données et ;
▪ la séquence simplifiée étant une séquence traduite où chaque acide aminé de la séquence initiale des CDRs est remplacé par un code représentant une catégorie d'acides aminés, les catégories étant basées sur les caractéristiques physico-chimiques des acides aminés, le nombre de catégories étant compris entre 4 et 10 ; et les catégories étant choisies parmi la taille des acides aminés, leur hydrophobicité, leur polarité, leur charge, leur caractère aromatique et des combinaisons de ces caractéristiques ;
▪ la structure secondaire étant une séquence traduite où chaque acide aminé de la séquence initiale des CDRs est remplacé par un code représentant un type de structure secondaire, le nombre de types de structure secondaire étant compris entre 3 et 8 et les types de structure secondaire sont choisis parmi une hélice 3₁₀, une hélice α, une hélice π, un coude fermé, un brin β, un résidu dans un pont β, un coude ou une absence de structure secondaire ;
- la fourniture d'une séquence simplifiée et une structure secondaire des CDRs CDR1, CDR2 et CDR3 pour un domaine variable d'un anticorps de référence ;
- le calcul d'un score de similitude entre le domaine variable de l'anticorps de référence et un domaine variable d'un anticorps test de la base de données sur la base de la séquence simplifiée de leurs CDRs et de la structure secondaire de leurs CDRs par identification de sous-séquences communes aux CDRs de l'anticorps de référence et de l'anticorps test ; et
- la sélection de l'anticorps test si le score de similitude est tel qu'il prédit que l'anticorps test et l'anticorps de référence sont capables de se lier à la même cible, l'anticorps test sélectionné étant appelé anticorps similaire.

2. Méthode selon la revendication 1 ou méthode permettant d'identifier des anticorps capables de se lier à la même cible, la méthode comprenant :
- la fourniture d'une base de données comprenant la caractérisation d'au moins 100 anticorps, chaque anticorps étant **caractérisé par** une séquence simplifiée et une structure secondaire des CDRs (Région Déterminante Complémentaire) CDR1, CDR2 et CDR3 pour chaque domaine variable d'un anticorps de la base de données et ;
▪ la séquence simplifiée étant une séquence traduite où chaque acide aminé de la séquence initiale des CDRs est remplacé par un code représentant une catégorie d'acides aminés, les catégories étant basées sur les caractéristiques physico-chimiques des acides aminés, le nombre de catégories étant compris entre 4 et 10 ; et les catégories étant choisies parmi la taille des acides aminés, leur hydrophobicité, leur polarité, leur charge, leur caractère aromatique et des combinaisons de ces caractéristiques ;
▪ la structure secondaire étant une séquence traduite où chaque acide aminé de la séquence initiale des CDRs est remplacé par un code représentant un type de structure secondaire, le nombre de types de structure secondaire étant compris entre 3 et 8 et les types de structure secondaire sont choisis parmi une hélice 3₁₀, une hélice α, une hélice π, un coude fermé, un brin β, un résidu dans un pont β, un coude ou une absence de structure secondaire ;
- la fourniture d'une séquence simplifiée et une structure secondaire des CDRs CDR1, CDR2 et CDR3 pour un domaine variable d'un anticorps de référence ;
- le calcul d'un score de similitude entre le domaine variable de l'anticorps de référence et un domaine variable d'un anticorps test de la base de données sur la base de la séquence simplifiée de leurs CDRs et de la structure secondaire de leurs CDRs par identification de sous-séquences communes aux CDRs de l'anticorps de référence et de l'anticorps test ; et
la sélection de l'anticorps test si le score de similitude est tel qu'il prédit que l'anticorps test et l'anticorps de référence sont capables de se lier à la même cible, l'anticorps test sélectionné étant appelé anticorps similaire,
**caractérisée en ce que** la méthode comprend en outre une étape de validation *in vitro* de la capacité de liaison de l'anticorps similaire à la cible de l'anticorps de référence ou de l'anticorps de référence à la cible de l'anticorps similaire.

3. Méthode selon la revendication 1 ou 2, **caractérisée en ce que** les catégories choisies sont
La petite taille pour les acides aminés A, G, S, T, C et P ;
Le caractère Aromatique pour les acides aminés Y, F et W ;
L'hydrophobicité pour les acides aminés I, L, F, M et V ;
La Polarité pour les acides aminés N et Q;
La charge positive pour les acides aminés H, K et R ; et
La charge négative pour les acides aminés D et E.

4. Méthode selon l'une quelconque des revendications 1-3, **caractérisée en ce que** les types de structure secondaire choisis sont :
- hélice 3₁₀ ;
- hélice α ;
- hélice π;
- coude fermé;
- brin β;
- résidu dans un pont β;
- coude; et
- une absence de structure secondaire.

5. Méthode selon la revendication 2, comprenant une étape de validation *in vitro* de la capacité de liaison de l'anticorps similaire à la cible de l'anticorps de référence.

6. Méthode selon la revendication 5, dans laquelle l'étape de validation comprend la fourniture ou la production de l'anticorps similaire, sa mise en contact avec la cible de l'anticorps de référence, et la mesure de la liaison entre l'anticorps similaire et la cible de l'anticorps de référence.

7. Méthode selon l'une quelconque des revendications 5-6, dans laquelle les CDR1, CDR2 et CDR3 sont greffés dans un squelette de nano-anticorps.

8. Méthode selon l'une quelconque des revendications 5-7, **caractérisée en ce que** l'anticorps similaire est sélectionné si la capacité de liaison de l'anticorps similaire à la cible de l'anticorps de référence est validée *in vitro.*

9. Méthode selon la revendication 2, comprenant une étape de validation *in vitro* de la capacité de liaison de l'anticorps de référence à la cible de l'anticorps similaire.

10. Méthode selon la revendication 9, dans laquelle l'étape de validation comprend la production ou la fourniture de l'anticorps de référence, sa mise en contact avec la cible de l'anticorps similaire, et la mesure de la liaison entre l'anticorps de référence et la cible de l'anticorps similaire.

11. Méthode selon l'une quelconque des revendications 9-10, **caractérisée en ce que** l'anticorps de référence est sélectionné si la capacité de liaison de l'anticorps de référence à la cible de l'anticorps similaire est validée *in vitro.*

12. Méthode selon l'une quelconque des revendications 1-11, **caractérisée en ce que** les anticorps sont des nano-anticorps.

## Patentansprüche

1. Computerimplementiertes Verfahren zur Identifizierung von Antikörpern, die an dasselbe Ziel binden können, wobei das Verfahren Folgendes umfasst:
- die Bereitstellung einer Datenbank, die die Charakterisierung von mindestens 100 Antikörpern umfasst, wobei jeder Antikörper durch eine vereinfachte Sequenz und eine Sekundärstruktur der CDRs (Complementary Determining Region) CDR1, CDR2 und CDR3 für jede variable Domäne eines Antikörpers aus der Datenbank gekennzeichnet ist und;
▪ die vereinfachte Sequenz eine translatierte Sequenz ist, in der jede Aminosäure der Anfangssequenz der CDRs durch einen Code ersetzt ist, der eine Kategorie von Aminosäuren darstellt, wobei die Kategorien auf den physikalisch-chemischen Eigenschaften der Aminosäuren basiert, wobei die Anzahl der Kategorien zwischen 4 und 10 liegt; und wobei die Kategorien ausgewählt sind aus der Größe der Aminosäuren, ihrer Hydrophobizität, ihrer Polarität, ihrer Ladung, ihrem aromatischen Charakter und Kombinationen dieser Eigenschaften;
▪ die Sekundärstruktur eine translatierte Sequenz ist, in der jede Aminosäure der Anfangssequenz der CDRs durch einen Code ersetzt ist, der einen Sekundärstrukturtyp darstellt, wobei die Anzahl der Sekundärstrukturtypen zwischen 3 und 8 liegt und die Sekundärstrukturtypen zwischen 3 und 8 liegen, ausgewählt aus einer 3₁₀ Helix; einer α Helix; einer π Helix; einer kurzen Schleife (tight turn); einem β Strang; einem Rest in einer β Brücke; einer Schleife (turn); oder dem Fehlen einer Sekundärstruktur;
- die Bereitstellung einer vereinfachten Sequenz und einer Sekundärstruktur der CDRs CDR1, CDR2 und CDR3 für eine variable Domäne eines Referenzantikörpers;
- die Berechnung eines Ähnlichkeits-Scores zwischen der variablen Domäne des Referenzantikörpers und einer variablen Domäne eines Testantikörpers aus der Datenbank auf der Grundlage der vereinfachten Sequenz ihrer CDRs und der Sekundärstruktur ihrer CDRs durch Identifizierung gemeinsamer Teilsequenzen der CDRs des Referenzantikörpers und des Testantikörpers; und
- die Auswahl des Testantikörpers, wenn der Ähnlichkeits-Score so ist, dass er vorhersagt, dass der Testantikörper und der Referenzantikörper in der Lage sind, an dasselbe Ziel zu binden, wobei der ausgewählte Testantikörper als ähnlicher Antikörper bezeichnet wird.

2. Verfahren nach Anspruch 1 oder ein Verfahren zum Identifizieren von Antikörpern, die an dasselbe Ziel binden können, wobei das Verfahren umfasst:
- die Bereitstellung einer Datenbank, die die Charakterisierung von mindestens 100 Antikörpern umfasst, wobei jeder Antikörper durch eine vereinfachte Sequenz und eine Sekundärstruktur der CDRs (Complementary Determining Region) CDR1, CDR2 und CDR3 für jede variable Domäne eines Antikörpers aus der Datenbank gekennzeichnet ist und;
▪ die vereinfachte Sequenz eine translatierte Sequenz ist, in der jede Aminosäure der Anfangssequenz der CDRs durch einen Code ersetzt ist, der eine Kategorie von Aminosäuren darstellt, wobei die Kategorien auf den physikalisch-chemischen Eigenschaften der Aminosäuren basiert, wobei die Anzahl der Kategorien, zwischen 4 und 10 liegt; und wobei die Kategorien ausgewählt sind aus der Größe der Aminosäuren, ihrer Hydrophobizität, ihrer Polarität, ihrer Ladung, ihrem aromatischen Charakter und Kombinationen dieser Eigenschaften;
▪ die Sekundärstruktur eine translatierte Sequenz ist, in der jede Aminosäure der Anfangssequenz der CDRs durch einen Code ersetzt ist, der einen Sekundärstrukturtyp darstellt, wobei die Anzahl der Sekundärstrukturtypen zwischen 3 und 8 liegt und die Sekundärstrukturtypen zwischen 3 und 8 liegen ausgewählt aus einer 3₁₀ Helix; einer α Helix; einer π Helix; einer kurzen Schleife (tight turn); einem β Strang; einem Rest in einer β Brücke; einer Schleife (turn); oder dem Fehlen einer Sekundärstruktur;
- die Bereitstellung einer vereinfachten Sequenz und einer Sekundärstruktur der CDRs CDR1, CDR2 und CDR3 für eine variable Domäne eines Referenzantikörpers;
- die Berechnung eines Ähnlichkeits-Scores zwischen der variablen Domäne des Referenzantikörpers und einer variablen Domäne eines Testantikörpers aus der Datenbank auf der Grundlage der vereinfachten Sequenz ihrer CDRs und der Sekundärstruktur ihrer CDRs durch Identifizierung gemeinsamer Teilsequenzen der CDRs des Referenzantikörpers und des Testantikörpers; und
die Auswahl des Testantikörpers, wenn der Ähnlichkeit-Score so ist, dass er vorhersagt, dass der Testantikörper und der Referenzantikörper in der Lage sind, an dasselbe Ziel zu binden, wobei der ausgewählte Testantikörper als ähnlicher Antikörper bezeichnet wird,
**dadurch gekennzeichnet, dass** das Verfahren ferner einen In-vitro-Validierungsschritt der Bindungskapazität des ähnlichen Antikörpers an das Ziel des Referenzantikörpers oder des Referenzantikörpers an das Ziel des ähnlichen Antikörpers umfasst.

3. Verfahren nach Anspruch 1 oder 2, **dadurch gekennzeichnet, dass** die gewählten Kategorien sind
die geringe Größe für die Aminosäuren A, G, S, T, C und P;
der aromatische Charakter für die Aminosäuren Y, F und W;
die Hydrophobizität für die Aminosäuren I, L, F, M und V;
die Polarität für die Aminosäuren N- und Q;
die positive Ladung für die Aminosäuren H, K und R; und
die negative Ladung für die Aminosäuren D und E.

4. Verfahren nach einem der Ansprüche 1-3, **dadurch gekennzeichnet, dass** die gewählten Arten von Sekundärstrukturen sind:
- 3₁₀ Helix;
- α Helix;
- π Helix;
- kurze Schleife (tight turn);
- β Strang;
- Rest in einer β Brücke;
- Schleife (turn); und
- das Fehlen einer Sekundärstruktur.

5. Verfahren nach Anspruch 2, umfassend einen In-vitro-Validierungsschritt der Bindungskapazität des ähnlichen Antikörpers an das Ziel des Referenzantikörpers.

6. Verfahren nach Anspruch 5, bei dem der Validierungsschritt das Bereitstellen oder Herstellen des ähnlichen Antikörpers, das Inkontaktbringen mit dem Ziel des Referenzantikörpers und das Messen der Bindung zwischen dem ähnlichen Antikörper und dem Ziel des Referenzantikörpers umfasst

7. Verfahren nach einem der Ansprüche 5-6, bei dem CDR1, CDR2 und CDR3 in das Skelett eines Nano-Antikörpers transplantiert werden.

8. Verfahren nach einem der Ansprüche 5-7, **dadurch gekennzeichnet, dass** der ähnliche Antikörper ausgewählt wird, wenn die Bindungskapazität des ähnlichen Antikörpers an das Ziel des Referenzantikörpers *in vitro* validiert ist.

9. Verfahren nach Anspruch 2, umfassend einen *In-vitro-*Validierungsschritt der Bindungskapazität des Referenzantikörpers an das Ziel des ähnlichen Antikörpers.

10. Verfahren nach Anspruch 9, bei dem der Validierungsschritt das Herstellen oder Bereitstellen des Referenzantikörpers, das Inkontaktbringen mit dem Ziel des ähnlichen Antikörpers und das Messen der Bindung zwischen dem Referenzantikörper und dem Ziel des ähnlichen Antikörpers umfasst.

11. Verfahren nach einem der Ansprüche 9-10, **dadurch gekennzeichnet, dass** der Referenzantikörper ausgewählt wird, wenn die Bindungskapazität des Referenzantikörpers an das Ziel des ähnlichen Antikörpers *in vitro* validiert ist.

12. Verfahren nach einem der Ansprüche 1-11, **dadurch gekennzeichnet, dass** die Antikörper Nano-Antikörper sind.

## Claims

1. Method, carried out by computer, making it possible to identify antibodies capable of binding to the same target, the method comprising:
- providing a database comprising the characterization of at least 100 antibodies, each antibody being **characterized by** a simplified sequence and a secondary structure of the CDRs (complementarity-determining regions) CDR1, CDR2 and CDR3 for each variable domain of an antibody of the database, and;
▪ the simplified sequence being a translated sequence in which each amino acid of the initial sequence of the CDRs is replaced by a code representing a category of amino acids, the categories being based on the physicochemical characteristics of the amino acids, the number of categories being between 4 and 10; and the categories being chosen from the size of the amino acids, their hydrophobicity, their polarity, their charge, their aromatic nature and combinations of these characteristics;
▪ the secondary structure being a translated sequence in which each amino acid of the initial sequence of the CDRs is replaced by a code representing a type of secondary structure, the number of types of secondary structure being between 3 and 8 and the types of secondary structure being chosen from a 3₁₀ helix, an α helix, a π helix, a tight turn, a β strand, a residue in a β-bridge, a turn or an absence of secondary structure;
- providing a simplified sequence and a secondary structure of the CDRs CDR1, CDR2 and CDR3 for a variable domain of a reference antibody;
- calculating a score for similarity between the variable domain of the reference antibody and a variable domain of a test antibody from the database based on the simplified sequence of their CDRs and on the secondary structure of their CDRs by identifying subsequences common to the CDRs of the reference antibody and of the test antibody; and
- selecting the test antibody if the similarity score is such that it predicts that the test antibody and the reference antibody are capable of binding to the same target, the test antibody selected being referred to as similar antibody.

2. Method according to Claim 1 or method to identify antibodies capable of binding to the same target, the method comprising:
- providing a database comprising the characterization of at least 100 antibodies, each antibody being **characterized by** a simplified sequence and a secondary structure of the CDRs (complementarity-determining regions) CDR1, CDR2 and CDR3 for each variable domain of an antibody of the database, and;
▪ the simplified sequence being a translated sequence in which each amino acid of the initial sequence of the CDRs is replaced by a code representing a category of amino acids, the categories being based on the physicochemical characteristics of the amino acids, the number of categories being between 4 and 10; and the categories being chosen from the size of the amino acids, their hydrophobicity, their polarity, their charge, their aromatic nature and combinations of these characteristics;
▪ the secondary structure being a translated sequence in which each amino acid of the initial sequence of the CDRs is replaced by a code representing a type of secondary structure, the number of types of secondary structure being between 3 and 8 and the types of secondary structure being chosen from a 3₁₀ helix, an α helix, a π helix, a tight turn, a β strand, a residue in a β-bridge, a turn or an absence of secondary structure;
- providing a simplified sequence and a secondary structure of the CDRs CDR1, CDR2 and CDR3 for a variable domain of a reference antibody;
- calculating a score for similarity between the variable domain of the reference antibody and a variable domain of a test antibody from the database based on the simplified sequence of their CDRs and on the secondary structure of their CDRs by identifying subsequences common to the CDRs of the reference antibody and of the test antibody; and
selecting the test antibody if the similarity score is such that it predicts that the test antibody and the reference antibody are capable of binding to the same target, the test antibody selected being referred to as similar antibody,
**characterized in that** the method also comprises a step of *in vitro* validation of the binding capacity of the similar antibody to the target of the reference antibody or of the reference antibody to the target of the similar antibody.

3. Method according to Claim 1 or 2, **characterized in that** the categories chosen are
small size for the amino acids A, G, S, T, C and P;
aromatic nature for the amino acids Y, F and W;
hydrophobicity for the amino acids I, L, F, M and V;
polarity for the amino acids N and Q;
positive charge for the amino acids H, K and R; and
negative charge for the amino acids D and E.

4. Method according to any one of Claims 1-3, **characterized in that** the types of secondary structure chosen are:
- 3₁₀ helix;
- α helix;
- π helix;
- tight turn;
- β strand;
- residue in a β-bridge;
- turn; and
- absence of secondary structure.

5. Method according to Claim 2, comprising a step of *in vitro* validation of the binding capacity of the similar antibody to the target of the reference antibody.

6. Method according to Claim 5, wherein the validation step comprises providing or producing the similar antibody, bringing it into contact with the target of the reference antibody, and measuring the binding between the similar antibody and the target of the reference antibody.

7. Method according to any one of Claims 5-6, wherein the CDR1, CDR2 and CDR3 are grafted into a nano-antibody backbone.

8. Method according to any one of Claims 5-7, **characterized in that** the similar antibody is selected if the binding capacity of the similar antibody to the target of the reference antibody is validated *in vitro.*

9. Method according to Claim 2, comprising a step of *in vitro* validation of the binding capacity of the reference antibody to the target of the similar antibody.

10. Method according to Claim 9, wherein the validation step comprises producing or providing the reference antibody, bringing it into contact with the target of the similar antibody, and measuring the binding between the reference antibody and the target of the similar antibody.

11. Method according to any one of Claims 9-10, **characterized in that** the reference antibody is selected if the binding capacity of the reference antibody to the target of the similar antibody is validated *in vitro.*

12. Method according to any one of Claims 1-11, **characterized in that** the antibodies are nano-antibodies.
